# EUROPÄISCHE PATENTSCHRIFT

(11) **EP 3 377 604 B1**
(45) Veröffentlichungstag und Bekanntmachung des Hinweises auf die Patenterteilung: **04.05.2022**
(21) Anmeldenummer: 16797906.1
(22) Anmeldetag: 17.11.2016
(51) Int. Cl.: C12M 1/00, C12M 1/02

(54) **ANLAGE ZUR AUFZUCHT UND REPRODUKTION VON MIKROORGANISMEN**
SYSTEM FOR GROWING AND REPRODUCING MICROORGANISMS
INSTALLATION POUR LA CULTURE ET LA REPRODUCTION DE MICRO-ORGANISMES

(30) Priorität: 20.11.2015 DE 102015222932
(43) Veröffentlichungstag der Anmeldung: 26.09.2018
(73) Patentinhaber: Alga Pangea GmbH, 6345 Kössen (AT)
(72) Erfinder: SCHUSTER, Jürgen, 8857 Siebnen (CH); ITTERMANN, Ralf, 99842 Ruhla (DE)
(74) Vertreter: Liedtke & Partner Patentanwälte
(86) Internationale Anmeldenummer: PCT/EP2016/077983
(87) Internationale Veröffentlichungsnummer: WO 2017/085177

(56) Entgegenhaltungen:
- EP-A1- 2 584 030
- EP-B1- 1 326 959
- DE-A1-102008 026 829
- DE-A1-102009 020 527
- DE-A1-102009 021 059
- DE-A1-102010 060 420
- DE-A1-102013 114 925
- DE-C1- 4 411 486
- US-A1- 2012 270 304

## Beschreibung

Die Erfindung betrifft eine Anlage zur Aufzucht und Reproduktion von Mikroorganismen.

Aus dem Stand der Technik ist die Aufzucht und Reproduktion von Mikroorganismen, z. B. Algen, mittels Beckenanlagen bekannt. Derartige Beckenanlagen weisen beispielsweise ein durch Trennwände gebildetes Vertikalmäandersystem auf, wobei eine Nährstoffsuspension in die Beckenanlage gefüllt wird und diese durchströmt.

Ein die Aufzucht von Mikroorganismen wesentlich beeinflussender Faktor ist eine Temperatur der Nährstoffsuspension. Die Temperatur bewirkt eine Basisaktivierung biologischer Systeme in den Zellen der Mikroorganismen, welche insbesondere die Aufnahme von Kohlenstoffdioxid, Photonen und Nährstoffe in Form organischer Düngematerialien umfassen. Wird eine ideale Temperatur der Nährstoffsuspension erreicht, können die genannten biologischen Systeme optimiert werden. Die ideale Temperatur sowie eine Toleranzbreite abweichend von der idealen Temperatur sind je nach Art des Mikroorganismus unterschiedlich. Die Temperatur der Nährstoffsuspension wird dabei im Wesentlichen durch eine Wärmeentwicklung bei der Erzeugung künstlichen Lichts zur Beleuchtung der Nährstoffsuspension sowie durch eine Wärmeentwicklung bei einer Photosynthese in den Zellen der Mikroorganismen beeinflusst. Daraus resultierend kann die reale Temperatur stark von der idealen Temperatur abweichen, wodurch ein Klima in der Beckenanlage zur Aufzucht der Mikroorganismen nicht mehr optimal ist.

DE 10 2008 026 829 A1 offenbart eine Anlage zur Aufzucht und Reproduktion von Mikroorganismen, bei der Licht in eine Nährstoffsuspension eingebracht wird. Des Weiteren wird in DE 41 34 813 A1 eine Einrichtung zur Kultivation von phototropen Mikroorganismen offenbart. DE 10 2013 109 747 A1 offenbart eine Vorrichtung zur Gewinnung von Phytoplankton.

Die DE 10 2009 020 527 A1 offenbart eine Vorrichtung zur Durchführung photochemischer Prozesse im Mikromaßstab, sowie die Verwendung der Vorrichtung zur Kultivierung von Photosynthese-betreibenden Zellen und/oder Mikroorganismen.

Eine weitere Vorrichtung zur Kultivierung photoautotropher Mikroorganismen ist aus der US 2012/0270304 A1 bekannt.

Der Erfindung liegt daher die Aufgabe zu Grunde, eine gegenüber dem Stand der Technik verbesserte Anlage zur Aufzucht und Reproduktion von Mikroorganismen anzugeben, wobei die Verlustwärme der Leuchteinheit effizient aus der Nährstoffsuspension abgeleitet werden kann.

Die Aufgabe wird erfindungsgemäß mit den in Anspruch 1 angegebenen Merkmalen gelöst.

Vorteilhafte Ausgestaltungen der Erfindung sind Gegenstand der Unteransprüche.

Eine erfindungsgemäße Anlage zur Aufzucht und Reproduktion von Mikroorganismen mit einer Beckenanlage umfasst eine Anzahl an Becken, wobei jeweils ein Becken ein durch Trennwände gebildetes, beleuchtbares Vertikalmäandersystem aufweist. In jeweils ein Becken ist dabei eine Nährstoffsuspension eingefüllt, wobei an einem unteren Ende der jeweiligen Trennwand eine Leuchteinheit angeordnet ist. Erfindungsgemäß ist zumindest eine Außenwand jeweils eines Beckens doppelwandig unter Ausbildung eines Hohlraums ausgebildet, wobei der Hohlraum von einem Temperiermedium zur Temperierung der Nährstoffsuspension durchströmbar ist, wobei zur Fixierung der Trennwände diese mittels Halterungen mit den Außenwänden kraftschlüssig oder formschlüssig oder in Kombination kraftschlüssig und formschlüssig verbunden sind und die Leuchteinheit zwischen zwei Halterungen jeweils einer Trennwand an dem unteren Ende dieser Trennwand angeordnet ist, wobei über die Halterungen eine enge thermische Kopplung zwischen dem Temperiermedium und der Leuchteinheit hergestellt ist, so dass eine Verlustwärme der Leuchteinheit mittels des Temperiermediums ableitbar ist.

Die derart ausgebildete Anlage ermöglicht eine optimale Temperierung der Nährstoffsuspension. Dadurch, dass die Nährstoffsuspension in ständigen Kontakt mit den Außenwänden ist, wird eine enge thermische Kopplung zwischen dem Temperiermedium und der Nährstoffsuspension erreicht. Dies verbessert die Aufzucht und Reproduktion der Mikroorganismen gegenüber dem Stand der Technik erheblich.

Die Außenwände sind vorzugsweise über ihre gesamte Flachseite doppelwandig ausgebildet, so dass eine homogene Temperierung der Nährstoffsuspension möglich ist. Als Temperiermedium wird beispielsweise eine Flüssigkeit, z. B.

Wasser, verwendet.

Gemäß einer Ausgestaltung der Erfindung umfassen die Außenwände jeweils ein wärmeleitfähiges Material, z. B. Edelstahl. Die Außenwände sind somit direkt thermisch mit der Nährstoffsuspension gekoppelt und übertragen eine Wärme der Nährstoffsuspension an das Temperiermedium.

Gemäß einer weiteren Ausgestaltung der Erfindung ist mindestens eine Temperaturerfassungseinheit zur Erfassung einer Temperatur innerhalb eines Beckens vorgesehen.

Beispielsweise ist die mindestens eine Temperaturerfassungseinheit zur Erfassung einer Temperatur der Nährstoffsuspension vorgesehen, wobei die mindestens eine Temperaturerfassungseinheit an einer Innenwandung einer Außenwand eines Beckens angeordnet ist. Die Temperatur der Nährstoffsuspension wird hierbei direkt erfasst, wobei vorzugsweise jedes Becken eine bestimmte Anzahl an Temperaturerfassungseinheiten aufweist.

Zusätzlich oder optional dazu ist die mindestens eine Temperaturerfassungseinheit zur Erfassung einer Temperatur des Temperiermediums vorgesehen. Dazu ist die Temperaturerfassungseinheit an einer Außenwandung der Außenwand, der Innenwandung der Außenwand oder im Temperiermedium selbst angeordnet. Darüber hinaus ist die mindestens eine Temperaturerfassungseinheit zusätzlich oder optional zur Erfassung einer Temperatur einer mit einer Leuchteinheit gekoppelten Kontaktfläche vorgesehen sein, so dass eine durch die Leuchteinheit erzeugte Verlustwärme erfassbar ist. Die Leuchteinheit umfasst beispielsweise eine Leuchtdiodenanordnung, die in den Trennwänden angeordnet ist. Hierbei ist die mindestens eine Temperaturerfassungseinheit beispielsweise an einer Trennwand angeordnet.

In einer weiteren Ausgestaltung der Erfindung ist ein Regelkreis vorgesehen, welchem als Eingangsgröße die von der mindestens einen Temperaturerfassungseinheit erfasste Temperatur des Temperiermediums zuführbar ist.

Der Regelkreis umfasst eine Regeleinheit, welche eine Temperatur des Temperiermediums in Abhängigkeit der von der mindestens einen Temperaturerfassungseinheit erfassten Temperatur regelt.

Des Weiteren umfasst der Regelkreis eine Datenverarbeitungseinheit zur Auswertung der von der mindestens einen Temperaturerfassungseinheit erfassten Temperatur. Die Datenverarbeitungseinheit ist dazu mit der Regeleinheit gekoppelt. Die Datenverarbeitungseinheit empfängt als Eingangsgröße die von der mindestens einen Temperaturerfassungseinheit erfasste Temperatur und speichert diese gegebenenfalls als Temperaturdaten ab. Hierbei ist der Regelkreis als ein digitaler Regelkreis ausgebildet. Alternativ kann der Regelkreis auch ohne Datenverarbeitungseinheit als ein analoger Regelkreis ausgebildet sein.

Darüber hinaus umfasst der Regelkreis ein Stellglied zur Einstellung einer von der Regeleinheit vorgegebenen Temperatur des Temperiermediums. Das Stellglied ist beispielsweise ein Temperierkörper oder ein separater Temperierkreislauf.

Ausführungsbeispiele der Erfindung werden im Folgenden anhand von Zeichnungen näher erläutert.

Darin zeigen:
- Figur 1: schematisch eine Draufsicht auf ein Ausführungsbeispiel einer Anlage zur Aufzucht und Reproduktion von Mikroorganismen mit vier Becken,
- Figur 2: schematisch ein Ausführungsbeispiel eines Beckens in Draufsicht,
- Figur 3: schematisch ein Ausführungsbeispiel eines Beckens in Schnittdarstellung,
- Figur 4: schematisch eine Seitenansicht eines Ausschnitts aus einem Becken,
- Figur 5: schematisch eine perspektivische Ansicht eines Ausschnitts aus einem Becken,
- Figur 6: schematisch eine Schnittdarstellung eines Ausschnitts aus einem Becken und
- Figur 7: schematisch ein Blockschaltbild eines Regelkreislaufs der Anlage.

Einander entsprechende Teile sind in allen Figuren mit den gleichen Bezugszeichen versehen.

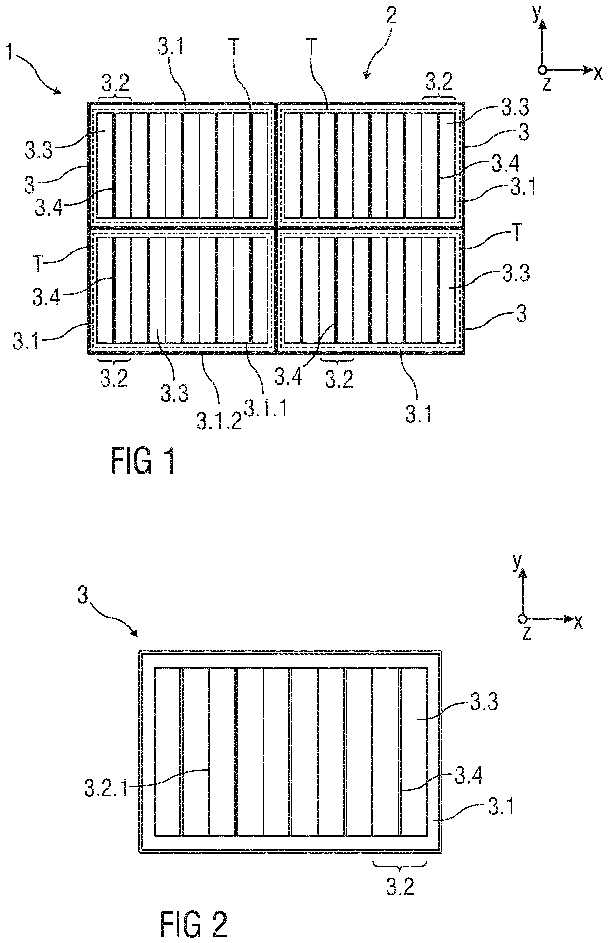

Zur besseren Veranschaulichung ist in allen Figuren 1 bis 6 ein dreidimensionales Koordinatensystem mit einer x-Achse, einer y-Achse und einer z-Achse dargestellt. Die z-Achse ist dabei eine vertikale Achse und die x-Achse und y-Achse sind jeweils eine horizontale Achse.

**Figur 1** zeigt schematisch ein Ausführungsbeispiel einer erfindungsgemäßen Anlage 1 zur Aufzucht und Reproduktion von Mikroorganismen, z. B. Algen, in Draufsicht.

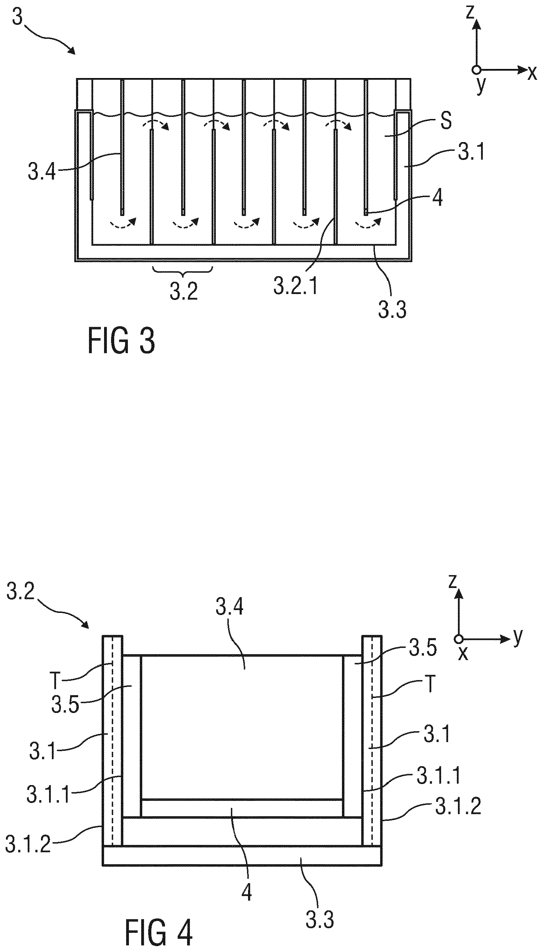

Die Anlage 1 umfasst eine Beckenanlage 2 mit beispielhaft vier Becken 3 und eine in der Beckenanlage 2 angeordnete Nährstoffsuspension S, die in den Figuren 3 und 4 dargestellt ist.

Die Becken 3 sind modulartig ausgebildet und weisen jeweils Außenwände 3.1 auf, die einen Innenraum der Becken 3 zur Aufnahme der Nährstoffsuspension S begrenzen. Innerhalb der Becken 3 sind jeweils einzelne in Richtung der x-Achse nebeneinander angeordnete Beckenzellen 3.2 mit einem im Wesentlichen u-förmigen Querschnitt angeordnet, die in Richtung der z-Achse nach oben offen ausgebildet sind.

Jede Beckenzelle 3.2 ist in Richtung der x-Achse durch zwei Seitenwände 3.2.1 begrenzt, die jeweils innerhalb des Beckens 3 zwischen zwei Außenwänden 3.1 in Richtung der y-Achse verlaufen und dabei von einem Beckenboden 3.3 aus in Richtung der z-Achse nach oben ragen. Die Seitenwände 3.2.1 benachbarter Beckenzellen 3.2 sind dabei derart dimensioniert, dass ein Überströmungsbereich der Nährstoffsuspension S von einer Beckenzelle 3.2 in die dazu benachbarte Beckenzelle 3.2 gebildet ist.

Des Weiteren ist in jede der Beckenzellen 3.2 eine Trennwand 3.4 eingetaucht, die zwischen den Seitenwänden 3.2.1 angeordnet ist und parallel zu diesen verläuft. Zum Beckenboden 3.3 sind die Trennwände 3.4 jeweils in Richtung der z-Achse beabstandet. Damit ist in jedem Becken 3 ein durch Trennwände 3.4 gebildetes Vertikalmäandersystem hergestellt, wobei eine im Wesentlichen vertikal gerichtete Strömung der Nährstoffsuspension S in der Beckenanlage 2 erreichbar ist.

Die Nährstoffsuspension S kann mittels einer Pumpe oder einer bewegbaren Platte in die Beckenanlage 2 eingebracht und dabei eine Strömung erzeugt werden, worauf im Rahmen dieser Anmeldung allerdings nicht näher eingegangen wird.

Die **Figuren 2** **und** **3** zeigen ein Becken 3 in verschiedenen Ansichten, wobei Figur 2 das Becken 3 in Draufsicht und Figur 3 das Becken 3 in einer Schnittdarstellung, insbesondere in einem Längsschnitt, zeigt.

Innerhalb eines Beckens 3 folgt die Nährstoffsuspension S im Wesentlichen einer vertikalen Strömung im Bereich zwischen einer Seitenwand 3.2.1 und einer Trennwand 3.4. In den Überströmungsbereichen und im Bereich zwischen dem Beckenboden 3.3 und einem dem Beckenboden 3.3 zugewandten Ende der Trennwand 3.4 wird die Strömung, wie durch Pfeile in Figur 3 gezeigt, umgelenkt, so dass hierdurch das Vertikalmäandersystem gebildet ist.

Die Trennwände 3.4 sind möglichst zentral in die jeweiligen Beckenzellen 3.2 eingetaucht, so dass die Abstände zwischen der Trennwand 3.4 und den jeweils benachbarten Seitenwänden in Richtung der x-Achse gleich sind. Erfindungsgemäß sind zur Fixierung der

Trennwände 3.4 diese beispielsweise mittels in Figur 4 beispielhaft gezeigten Halterungen 3.5 mit den Außenwänden 3.1 kraftschlüssig oder formschlüssig oder in Kombination kraftschlüssig und formschlüssig verbindbar. verbunden.

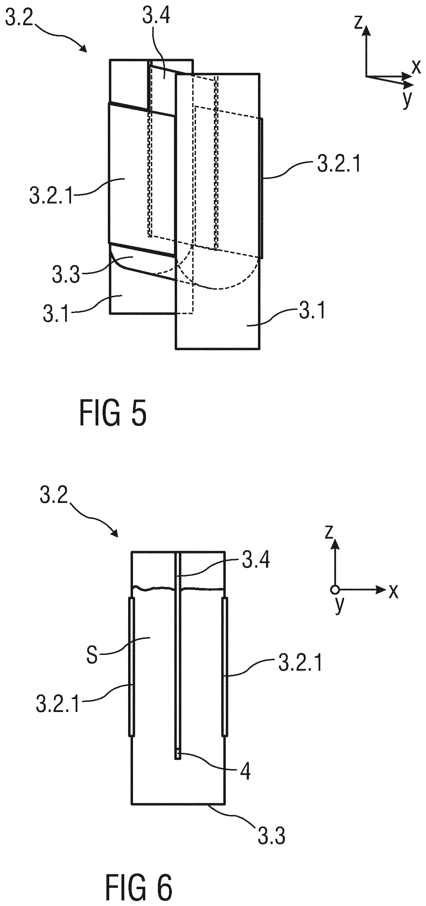

Zur besseren Veranschaulichung der Fixierung der Trennwände 3.4 zeigt **Figur 4** eine Seitenansicht eines Ausschnitts aus einem Becken 3. Die **Figuren 5 und 6** zeigen jeweils eine einzelne Beckenzelle 3.2, wobei Figur 5 die Beckenzelle 3.2 perspektivisch und Figur 6 in einer Seitenansicht zeigt.

Die Halterungen 3.5 verlaufen in Richtung der z-Achse parallel zu den Außenwänden 3.1 und weisen beispielsweise Führungsschlitze auf, welche die Kanten der Trennwände 3.4 formschlüssig aufnehmen, so dass diese in die jeweiligen Beckenzellen 3.2 einschiebbar sind.

Des Weiteren ist eine Leuchteinheit 4 gezeigt, die zum Einbringen von Licht und Wärme in die Nährstoffsuspension S vorgesehen ist. Die Leuchteinheit 4 ist an einem in Betrachtungsrichtung unteren Ende der Trennwand 3.4 angeordnet und umfasst eine Anzahl an Lichtquellen, z. B. Leuchtdioden oder Glühbirnen oder andere geeignete Leuchtelemente.

Das von der Leuchteinheit 4 erzeugte Licht wird über lichtdurchlässige Bereiche in der Trennwand 3.4 an die Nährstoffsuspension S abgegeben. Die lichtdurchlässigen Bereiche können über die gesamte Umfangsfläche der Trennwände 3.4 ausgebildet sein, wobei die Trennwand 3.4 hierbei vollständig oder abschnittsweise aus Milchglas oder einem transparenten Kunststoff gebildet ist.

Zusätzlich dazu kann die Nährstoffsuspension S mittels Sonnenlicht beleuchtet werden.

Wie bereits eingangs beschrieben, ist die Temperatur der Nährstoffsuspension S neben der Beleuchtung ein wesentlicher die Aufzucht von Mikroorganismen beeinflussender Faktor.

Die Temperatur der Nährstoffsuspension S wird dabei einerseits durch eine bei der Beleuchtung der Nährstoffsuspension S entstehende Wärme, insbesondere eine Verlustwärme der Leuchtelemente, und andererseits durch eine bei einer Photosynthese in den Zellen der Mikroorganismen entstehende Wärme beeinflusst.

Da ein maximaler Wachstums- und Zellteilungsprozess bei der Aufzucht von Mikroorganismen wünschenswert ist, wird der Lichteintrag bei der Beleuchtung intensiviert. Aus einer höheren Beleuchtungsintensität resultiert allerdings auch eine höhere Wärmeentwicklung, da aufgrund der räumlich nahen Anordnung der Leuchteinheit 4 zur Nährstoffsuspension S auch eine höhere Verlustwärme der Leuchtelemente eine weitere Erwärmung der Nährstoffsuspension S bedingt. Dies kann zu einem unerwünschten Abweichen der Temperatur der Nährstoffsuspension S von einem Idealwert oder einem Toleranzbereich führen.

Im Rahmen der Erfindung sind daher für eine optimale Temperierung der Nährstoffsuspension S die Außenwände 3.1 der Becken 3 doppelwandig ausgebildet. Dabei können die Außenwände 3.1 bereichsweise, einzelne Außenwände 3.1 oder alle Außenwände 3.1 doppelwandig ausgebildet sein. Denkbar ist auch, die Seitenwände 3.2.1 und den Beckenboden 3.3 doppelwandig auszubilden.

Die Außenwände 3.1 eines Beckens 3 weisen somit eine Innenwandung 3.1.1 und eine Außenwandung 3.1.2 auf, zwischen denen ein Hohlraum ausgebildet ist, welcher von einem Temperiermedium T durchströmbar ist.

Das Temperiermedium T ist vorzugsweise eine Flüssigkeit, z. B. eine Salzlösung und durchströmt die doppelwandige Außenwand 3.1 in Richtung der x-Achse, y-Achse und/oder z-Achse. Denkbar ist auch, in dem zwischen der Außenwandung 3.1.2 und Innenwandung 3.1.1 gebildeten Hohlraum Kanal strukturen zur Strömungsführung anzuordnen.

Vorzugsweise sind die Außenwände 3.1 jeweils aus einem gut wärmeleitfähigen Material, z. B. Edelstahl gebildet, so dass eine ideale Wärmeübertragung zwischen den Außenwänden 3.1 und dem Temperiermedium T sowie zwischen den Außenwänden 3.1 und der Nährstoffsuspension S und anderen die Außenwand 3.1 kontaktierenden Elementen möglich ist.

Dadurch, dass die Außenwände 3.1 in ständigem Kontakt mit der Nährstoffsuspension S sind, ist eine enge thermische Kopplung zwischen dem Temperiermedium T und der Nährstoffsuspension S möglich. Erfindungsgemäß ist die Halterungen 3.5 zudem eine enge thermische Kopplung zwischen dem Temperiermedium T und der Leuchteinheit 4 hergestellt, so dass eine Verlustwärme der Leuchteinheit 4 mittels des Temperiermediums T ableitbar ist.

Die Hohlräume benachbarter Außenwände 3.1 können dabei strömungstechnisch verbunden sein oder bilden jeweils einen separaten Hohlraum aus, welcher mit einer außerhalb der Beckenanlage 2 verlaufenden Temperierleitung (nicht dargestellt) verbindbar ist.

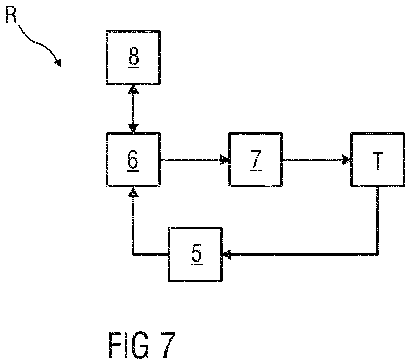

Zur Regelung einer Temperatur des Temperiermediums T ist ein in **Figur 7** schematisch dargestellter digitaler Regelkreis R vorgesehen.

Der Regelkreis R umfasst als zu regelnde Größe eine Temperatur des Temperiermediums T, mindestens eine Temperaturerfassungseinheit 5 zur mittelbaren oder unmittelbaren Erfassung einer Ist-Temperatur des Temperiermediums T sowie eine Regeleinheit 6 zur Regelung der Ist-Temperatur auf eine Soll-Temperatur und ein Stellglied 7, welches die von der Regeleinheit 6 vorgegebene Soll-Temperatur einstellt.

Die mindestens eine Temperaturerfassungseinheit 5 ist zur Erfassung einer Temperatur des Temperiermediums T vorgesehen, wobei diese an der Außenwandung 3.1.2 oder der Innenwandung 3.1.1 der Außenwand 3.1 oder im Temperiermedium T selbst angeordnet ist. Die mindestens eine Temperaturerfassungseinheit 5 ist dabei als ein bekannter Temperatursensor ausgebildet.

Alternativ kann die mindestens eine Temperaturerfassungseinheit 5 zur Erfassung einer Temperatur der Nährstoffsuspension S vorgesehen sein, wobei die mindestens eine Temperaturerfassungseinheit 5 an der Innenwandung 3.1.1 der Außenwand 3.1 angeordnet ist. Die Temperatur der Nährstoffsuspension S wird hierbei direkt erfasst, wobei vorzugsweise jedes Becken 3 eine bestimmte Anzahl an Temperaturerfassungseinheiten 5 aufweist.

Weiterhin kann zusätzlich oder optional mindestens eine Temperaturerfassungseinheit 5 zur Erfassung einer Temperatur einer mit der Leuchteinheit 4 gekoppelten Kontaktfläche vorgesehen sein, so dass eine durch die Leuchteinheit 4 erzeugte Abwärme erfassbar ist. Hierbei ist die mindestens eine Temperaturerfassungseinheit 5 beispielsweise an einer Trennwand 3.4 angeordnet.

Des Weiteren ist es möglich, dass im Becken 3 mehrere Temperaturerfassungseinheiten 5 an verschiedenen Stellen, insbesondere an den zuvor erwähnten Stellen im Becken 3 angeordnet sind, so dass mehrere Temperaturdaten erfasst werden.

Die erfassten Temperaturdaten dienen der Regeleinheit 6 als Eingangsgröße, welche diese mit einer Sollgröße vergleicht und entsprechend eine Stellgröße an das Stellglied 7, z. B. ein Wärmetauscher, übermittelt. Somit kann in Abhängigkeit auch mehrerer Eingangsgrößen die Temperatur des Temperiermediums T geregelt werden.

Zur Auswertung der Eingangsgrößen in der Regeleinheit 6 ist diese mit einer Datenverarbeitungseinheit 8 gekoppelt. Alternativ ist die Datenverarbeitungseinheit 8 in die Regeleinheit 6 integriert. Die Datenverarbeitungseinheit 8 speichert die Eingangsgrößen gegebenenfalls als Temperaturdaten ab.

### BEZUGSZEICHENLISTE

- 1: Anlage
- 2: Beckenanlage
- 3: Becken
- 3.1: Außenwand
- 3.1.1: Innenwandung
- 3.1.2: Außenwandung
- 3.2: Beckenzelle
- 3.2.1: Seitenwand
- 3.3: Beckenboden
- 3.4: Trennwand
- 3.5: Halterung
- 4: Leuchteinheit
- 5: Temperaturerfassungseinheit
- 6: Regeleinheit
- 7: Stellglied
- 8: Datenverarbeitungseinheit

- R: Regelkreis
- S: Nährstoffsuspension
- T: Temperiermedium

- x, y, z: Achse

## Patentansprüche

1. Anlage (1) zur Aufzucht und Reproduktion von Mikroorganismen mit einer Beckenanlage (2), umfassend eine Anzahl an Becken (3), wobei
- jeweils ein Becken (3) ein durch Trennwände (3.4) gebildetes, beleuchtbares Vertikalmäandersystem aufweist, und
- in jeweils einem Becken (3) eine Nährstoffsuspension (S) eingefüllt ist,
- wobei an einem unteren Ende der jeweiligen Trennwand (3.4) eine Leuchteinheit (4) angeordnet ist,
**dadurch gekennzeichnet, dass**
- zumindest eine Außenwand (3.1) jeweils eines Beckens (3) doppelwandig unter Ausbildung eines Hohlraums ausgebildet ist, und
- der Hohlraum von einem Temperiermedium (T) zur Temperierung der Nährstoffsuspension (S) durchströmbar ist,
- wobei zur Fixierung der Trennwände (3.4) diese mittels Halterungen (3.5) mit den Außenwänden (3.1) kraftschlüssig oder formschlüssig oder in Kombination kraftschlüssig und formschlüssig verbunden sind und die Leuchteinheit (4) zwischen zwei Halterungen (3.5) jeweils einer Trennwand (3.4) an dem unteren Ende dieser Trennwand (3.4) angeordnet ist,
wobei über die Halterungen (3.5) eine enge thermische Kopplung zwischen dem Temperiermedium (T) und der Leuchteinheit (4) hergestellt ist, so dass eine Verlustwärme der Leuchteinheit (4) mittels des Temperiermediums (T) ableitbar ist.

2. Anlage (1) nach Anspruch 1, wobei die doppelwandig ausgebildeten Außenwände (3.1) ein wärmeleitfähiges Material umfassen.

3. Anlage (1) nach Anspruch 1 oder 2, umfassend mindestens eine Temperaturerfassungseinheit (5) zur Erfassung einer Temperatur innerhalb eines Beckens (3).

4. Anlage (1) nach Anspruch 3, wobei die mindestens eine Temperaturerfassungseinheit (5) zur Erfassung einer Temperatur der Nährstoffsuspension (S) vorgesehen ist.

5. Anlage (1) nach Anspruch 3 oder 4, wobei die mindestens eine Temperaturerfassungseinheit (5) zur Erfassung einer Temperatur des Temperiermediums (T) vorgesehen ist.

6. Anlage (1) nach einem der Ansprüche 3 bis 5, wobei die mindestens eine Temperaturerfassungseinheit (5) zur Erfassung einer Temperatur einer mit der Leuchteinheit (4) gekoppelten Kontaktfläche vorgesehen ist.

7. Anlage (1) nach einem der Ansprüche 3 bis 6, umfassend einen Regelkreis (R), welchem als Eingangsgröße die von der mindestens einen Temperaturerfassungseinheit (5) erfasste Temperatur des Temperiermediums (T) zuführbar ist.

8. Anlage (1) nach Anspruch 7, wobei der Regelkreis (R) eine Regeleinheit (6) umfasst, welche eine Temperatur des Temperiermediums (T) in Abhängigkeit der von der mindestens einen Temperaturerfassungseinheit (5) erfassten Temperatur regelt.

9. Anlage (1) nach Anspruch 8, wobei
- der Regelkreis (R) eine Datenverarbeitungseinheit (8) zur Auswertung der von der mindestens einen Temperaturerfassungseinheit (5) erfassten Temperatur umfasst, und
- die Datenverarbeitungseinheit (8) mit der Regeleinheit (6) gekoppelt ist.

10. Anlage (1) nach Anspruch 8 oder 9, wobei der Regelkreis (R) ein Stellglied (7) zur Einstellung einer von der Regeleinheit (6) vorgegebenen Temperatur des Temperiermediums (T) umfasst.

## Claims

1. System (1) for growth and reproduction of microorganisms having a basin system (2), comprising a number of basins (3), wherein
- each basin (3) has an illuminable vertical meandering system formed by partition walls (3.4), and
- each basin (3) is filled with a nutrient suspension (S),
- wherein a lighting unit (4) is arranged on a lower end of each partition wall (3.4),
**characterized in that**
- at least one outer wall (3.1) of each basin (3) is double-walled to form a cavity, and
- a temperature-control medium (T) for temperature-control of the nutrient suspension (S) is flowable through the cavity,
- wherein, for fixation of the partition walls (3.4), they are connected by means of holders (3.5) to the outer walls (3.1) by means of a force-fit or form-fit or a combination of a force-fit and form-fit, and the lighting unit (4) is arranged between two holders (3.5) of each partition wall (3.4) on the lower end of said partition wall (3.4),
wherein tight thermal coupling between the temperature-control medium (T) and the lighting element (4) is established via the holders (3.5), such that heat loss from the lighting unit (4) is dissipatable by means of the temperature-control medium (T).

2. System (1) according to Claim 1, wherein the double-walled outer wall (3.1) comprises a thermally conductive material.

3. System (1) according to Claim 1 or 2, comprising at least one temperature measurement unit (5) for measurement of a temperature within a basin (3).

4. System (1) according to Claim 3, wherein the at least one temperature measurement unit (5) is intended for measurement of a temperature of the nutrient suspension (S).

5. System (1) according to Claim 3 or 4, wherein the at least one temperature measurement unit (5) is intended for measurement of a temperature of the temperature-control medium (T).

6. System (1) according to any of Claims 3 to 5, wherein the at least one temperature measurement unit (5) is intended for measurement of a temperature of a contact surface coupled to the lighting unit (4).

7. System (1) according to any of Claims 3 to 6, comprising a closed-loop control system (R) to which the temperature of the temperature-control medium (T) measured by the at least one temperature measurement unit (5) is feedable as an input variable.

8. System (1) according to Claim 7, wherein the closed-loop control system (R) comprises a control unit (6) which controls a temperature of the temperature-control medium (T) depending on the temperature measured by the at least one temperature measurement unit (5).

9. System (1) according to Claim 8, wherein
- the closed-loop control system (R) comprises a data processing unit (8) for evaluation of the temperature measured by the at least one temperature measurement unit (5), and
- the data processing unit (8) is coupled to the control unit (6).

10. System (1) according to Claim 8 or 9, wherein the closed-loop control system (R) comprises an actuator (7) for setting of a temperature of the temperature-control medium (T) specified by the control unit (6).

## Revendications

1. Système (1) de culture et de reproduction de microorganismes muni d'un système de cuvettes (2), comprenant un certain nombre de cuvettes (3),
- chaque cuvette (3) comportant un système de méandres verticaux qui peut être éclairé et qui est formé par des cloisons (3.4), et
- une suspension nutritive (S) étant versée dans chaque cuvette (3),
- une unité d'éclairage (4) étant disposée à une extrémité inférieure de la cloison (3.4) respective, **caractérisé en ce que**
- au moins une paroi extérieure (3.1) de chaque cuvette (3) est conçue comme une double paroi formant une cavité, et
- un milieu de régulation de température (T) pouvant s'écouler à travers la cavité afin de réguler la température de la suspension nutritive (S),
- pour fixer les cloisons (3.4), celles-ci étant reliées, en force ou par complémentarité de formes ou de manière combinée en force et par complémentarité de formes, aux parois extérieures (3.1) au moyen de supports (3.5) et l'unité d'éclairage (4) étant disposée entre deux supports (3.5) d'une cloison (3.4) à l'extrémité inférieure de cette cloison (3.4), les supports (3.5) réalisant un couplage thermique étroit entre le milieu de régulation de température (T) et l'unité d'éclairage (4) de sorte que la chaleur perdue de l'unité d'éclairage (4) puisse être déviée au moyen du milieu de régulation de température (T).

2. Système (1) selon la revendication 1, les parois extérieures (3.1) qui forment la double paroi comprenant un matériau thermoconducteur.

3. Système (1) selon la revendication 1 ou 2, comprenant au moins une unité de détection de température (5) destinée à détecter la température à l'intérieur d'une cuvette (3).

4. Système (1) selon la revendication 3, l'au moins une unité de détection de température (5) étant prévue pour détecter la température de la suspension nutritive (S).

5. Système (1) selon la revendication 3 ou 4, l'au moins une unité de détection de température (5) étant prévue pour détecter une température du milieu de régulation de température (T).

6. Système (1) selon l'une des revendications 3 à 5, l'au moins une unité de détection de température (5) étant prévue pour détecter la température d'une surface de contact couplée à l'unité d'éclairage (4).

7. Système (1) selon l'une des revendications 3 à 6, comprenant un circuit de régulation (R) auquel la température du milieu de régulation de température (T), qui est détectée par l'au moins une unité de détection de température (5), peut être fournie comme grandeur d'entrée.

8. Système (1) selon la revendication 7, le circuit de régulation (R) comprenant une unité de régulation (6) qui régule une température du milieu de régulation de température (T) en fonction de la température détectée par l'au moins une unité de détection de température (5).

9. Système (1) selon la revendication 8,
- le circuit de régulation (R) comprenant une unité de traitement de données (8) destinée à évaluer la température détectée par l'au moins une unité de détection de température (5), et
- l'unité de traitement de données (8) étant couplée à l'unité de commande (6).

10. Système (1) selon la revendication 8 ou 9, le circuit de régulation (R) comprenant un organe de réglage (7) destiné à régler une température du milieu de régulation de température (T) qui est spécifiée par l'unité de commande (6).
